(19) Europäisches Patentamt
European Patent Office
Office européen des brevets

(11) Numéro de publication : 0 306 408 B1

# (12) FASCICULE DE BREVET EUROPEEN

(45) Date de publication du fascicule du brevet : **18.03.92 Bulletin 92/12**

(51) Int. Cl.$^5$ : **C07D 487/04,** A61K 31/50, // (C07D487/04, 237:00, 235:00)

(21) Numéro de dépôt : **88402199.9**

(22) Date de dépôt : **01.09.88**

## (54) Imidazo[1,2b]pyridazines, procédé pour leur préparation et compositions pharmaceutiques les contenant.

(30) Priorité : **01.09.87 FR 8712167**

(43) Date de publication de la demande : **08.03.89 Bulletin 89/10**

(45) Mention de la délivrance du brevet : **18.03.92 Bulletin 92/12**

(84) Etats contractants désignés : **AT BE CH DE FR GB IT LI LU NL SE**

(56) Documents cités :
**GB-A- 1 135 893**
**US-A- 4 569 934**

(73) Titulaire : **SANOFI S.A.**
**40, Avenue George V**
**F-75008 Paris (FR)**

(72) Inventeur : **Bourguignon, Jean Jacques**
**2, rue du Feldwasser**
**F-67150 Hipsheim (FR)**
Inventeur : **Wermuth, Camille George**
**3, rue de la Côte d'azur**
**F-67100 Strasbourg (FR)**
Inventeur : **Worms, Paul**
**10, rue Devois**
**F-34980 Saint Gely du Fesc (FR)**

(74) Mandataire : **Gillard, Marie-Louise et al**
**Cabinet Beau de Loménie 55, Rue d'Amsterdam**
**F-75008 Paris (FR)**

EP 0 306 408 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention a pour objet des dérivés aminés de l'imidazo [1,2-b] pyridazine, un procédé pour leur préparation et des compositions pharmaceutiques contenant lesdites imidazo [1,2-b] pyridazines comme ingrédients actifs.

Plus précisément, suivant un premier aspect, la présente invention concerne donc des composés nouveaux répondant à la formule générale :

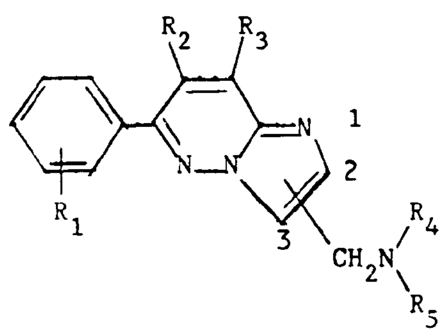

(I)

dans laquelle :

- $R_1$ représente l'hydrogène, un atome d'halogène, un groupe alcoxy en $C_1$ - $C_4$, un groupe alkyle en $C_1$ - $C_4$ occupant l'une des positions libres du noyau benzénique ;
- $R_2$ représente l'hydrogène, un groupe alkyle en $C_1$ - $C_4$ ou un groupe phényle ;
- $R_3$ désigne l'hydrogène, un groupe alkyle en $C_1$ - $C_4$ ou un groupe phényle ;
- Le groupe

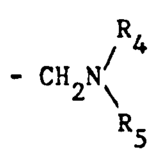

occupe la position 2 ou la position 3 du cycle et $R_4$ et $R_5$ considérés indépendemment représentent l'hydrogène ou un groupe alkyle en $C_1$ - $C_4$ ou encore $R_4$ et $R_5$ considérés ensemble avec l'atome d'azote auquel ils sont liés constituent un hétérocycle à 5 ou 6 chaînons contenant éventuellement un second hétéroatome et notamment les cycles pyrrolidine, pipéridine, morpholine, ainsi que les sels d'addition formés par les composés de formule (I) avec les acides minéraux ou organiques pharmaceutiquement acceptables.

Selon un second aspect, l'invention a pour objet un procédé de préparation des composés de formule (I) pouvant être représenté par le schéma suivant :

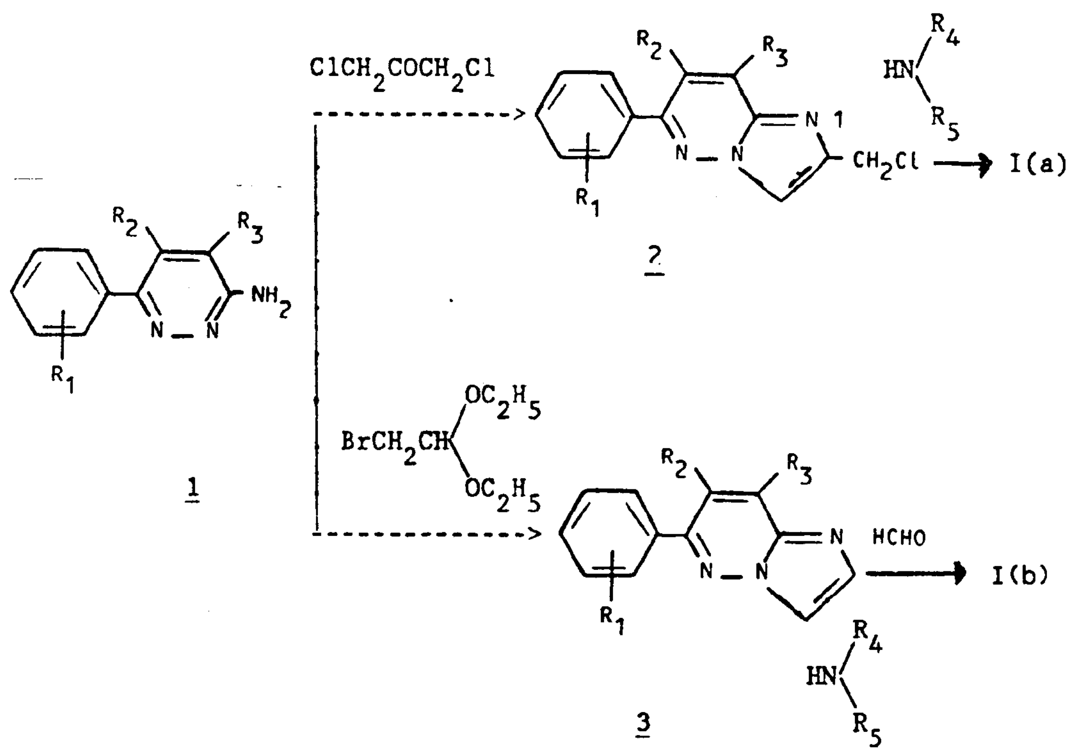


1 2 3

Les composés de formule (I) sont préparés à partir des amino-3 phényl-6 pyridazines convenablement substituées 1.

Lorsque le groupe

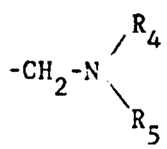

occupe la position 2 (composés Ia), on fait agir sur l'amine 1 la dichloro-1,3 acétone qui fournit le dérivé chlorométhylé 2. On opère par chauffage des réactifs, de préférence entre 70 et 110°C, en général pendant 12 à 15 heures, dans un solvant convenable tel que le dioxanne ou l'acétonitrile et on ajoute en fin de réaction, de l'acétate de sodium.

Par chauffage du dérivé chlorométhylé 2 avec l'amine

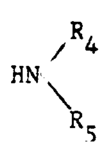

au sein d'un solvant inerte,
on obtient le composé I(a).

Celui-ci est éventuellement salifié selon une méthode connue en elle même.

Lorsque le groupe

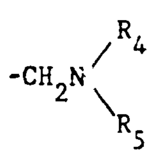

occupe la position 3 (composés Ib), on fait agir sur l'amine 1 le bromoacétaldéhyde qui conduit à l'imidazo pyridazine 3. On opère en solution dans un solvant convenable, par exemple un solvant protique, comme un alcool et spécialement l'éthanol ou un mélange alcool/eau, à une température modérée comprise entre 20 et 50°C.

Dans la pratique, le bromoacétaldéhyde est préparé par hydrolyse acide en milieu alcoolique d'un acétal tel que diéthylacétal correspondant, et la solution alcoolique ainsi obtenue est utilisée directement pour la réaction avec l'amine 1.

A partir de l'imidazo pyridazine 3, on obtient les composés I(b) par action du formol et de l'amine

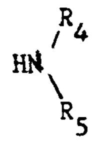

dans les conditions de la réaction de Mannich.

Les produits Ib ainsi obtenus sont éventuellement salifiés selon une méthode connue.

Les amino pyridazines 1 utilisés comme produits de départ sont des composés connus ou peuvent être préparés selon des procédés connus.

Les exemples suivants illustrent l'invention sans en limiter la portée :

## EXEMPLE 1

Dichlorhydrate de diéthylaminométhyl-2 méthyl-8 phényl-6 imidazo [1,2-b] pyridazine (SR 96052 A).
Ia : $R_1 = R_2 = H$ $R_3 = -CH_3$ $R_4 = R_5 = -C_2H_5$

A) Chlorométhyl-2 méthyl-8 phényl-6 imidazo [1,2-b] pyridazine.

On chauffe à reflux pendant 15 heures, la solution dans le dioxanne de 1,85 g d'amino-3 méthyl-4 phényl-6 pyridazine et 1,52 g de dichloro-1,3 acétone.

On ajoute 0,41 g d'acétate de sodium et porte à nouveau au reflux pendant 4 heures.

On évapore à siccité puis chromatographie le résidu sur colonne de silice. En éluant avec le mélange Hexane-acétate d'éthyle 7/3 vol/vol on obtient le produit attendu qui cristallise.
F : 124°C Rendement 66 %.

B) SR 96052 A.

On chauffe au reflux pendant 24 heures la solution de 0,65 g du dérivé chlorométhylé obtenu ci-dessus et 1,05 ml de diéthylamine dans 15 ml de tétrahydrofuranne.

On filtre le précipité de chlorhydrate de diéthylamine qu'on lave avec un peu de tétrahydrofuranne. On évapore le solvant et reprend le résidu dans l'eau. On extrait avec de l'acétate d'éthyle, sépare la phase organique et sèche la solution.

On évapore le solvant et chromatographie le résidu sur colonne de silice. On élue avec le mélange acétate d'éthyle-méthanol 98/2 vol/vol additionné de 2 % d'ammoniaque. On obtient le produit attendu sous forme d'huile.
Rendement 52 %.

### Dichlorhydrate

On dissout la base ci-dessus dans le minimum d'isopropanol chaud et ajoute 2,2 équivalents d'acide chlorhydrique concentré. Par refroidissement il se sépare des cristaux qu'on essore et lave avec l'éther isopropylique F : 154°C.

## EXEMPLES 2 à 9

A) Dérivés chlorométhylés.

En opérant comme dans l'exemple 1A, mais en faisant varier le dérivé aminé de départ, on obtient les dérivés chlorométhylés rassemblés dans le tableau 1.

TABLEAU 1

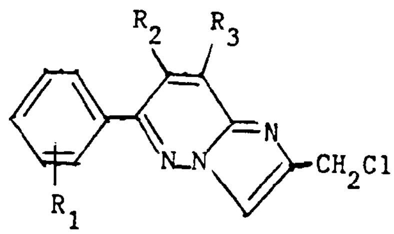

| $R_1$ | $R_2$ | $R_3$ | Constantes physiques |
|---|---|---|---|
| H | H | H | F : 140°C |
| H | H | $-C_6H_5$ | Chromatographié (1) |
| $3-OCH_3$ | H | $-CH_3$ | F : 125°C (1) |
| 4-Cl | H | H | F : 180°C (1) |
| 2-Cl | H | H | F : 128°C (1) |

(1) Pour ces produits la réaction a été effectuée dans l'acétonitrile au lieu du dioxanne.

B) A partir des dérivés chlorométhylés décrits dans l'exemple 1A et dans le tableau 1, on obtient suivant la technique décrite dans l'exemple 1B et en faisant varier l'amine utilisée, les composés Ia réunis dans le tableau 2.

TABLEAU 2

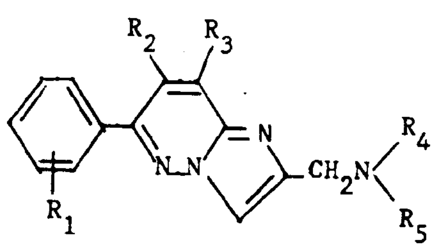

| Ex n° | N° Code SR | $R_1$ | $R_2$ | $R_3$ | 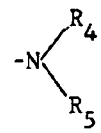 | Base ou sel Point de fusion |
|---|---|---|---|---|---|---|
| 2 | 95775 A | H | H | $-CH_3$ | 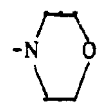 | Base : 112°C Dichlorhydrate, 0,5$H_2O$ : > 260°C |
| 3 | 96069 A | H | H | H | 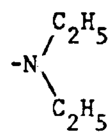 | Dichlorhydrate, 1,5$H_2O$ : 222°C |
| 4 | 95842 A | H | H | H | 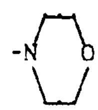 | Base : 100°C Dichlorhydrate : 240°C (éthanol) |
| 5 | 96045 A | H | H | $-C_6H_5$ | " " | Dichlorhydrate : > 300°C (éthanol) |
| 6 | 96072 A | 3-$OCH_3$ | H | $-CH_3$ | " " | Dichlorhydrate,1$H_2O$ : 217°C (éthanol) |
| 7 | 96089 A | 4-Cl | H | H | " " | Monochlorhydrate,0,5 $H_2O$ : 262°C (éthanol) |
| 8 | 96095 A | 3-$OCH_3$ | H | $-CH_3$ | 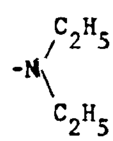 | Dichlorhydrate,2$H_2O$ |
| 9 | 96110 A | 2-Cl | H | H | 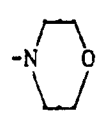 | Monochlorhydrate : 250°C (éthanol) |

EXEMPLE 10

Dichlorhydrate de méthyl-8 morpholinométhyl-3 phényl-6 imidazo [1,2-b] pyridazine (SR 95950 A).

Ib : $R_1 = R_2 = H$ $R_3 = -CH_3$ 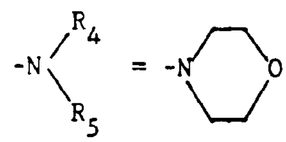

A) Méthyl-8 phényl-6 imidazo [1,2-b] pyridazine.

On chauffe au reflux pendant 2 heures, le mélange de 3 g de bromacétaldéhyde diéthylacétal, 0,75 ml d'acide bromhydrique concentré et 0,75 ml d'eau. On verse dans 25 ml d'éthanol et neutralise avec du bicarbonate de sodium solide. On filtre le précipité qu'on lave avec un peu d'éthanol.

Au filtrat (solution de bromacétaldéhyde), on ajoute 1,2 g d'amino-3 méthyl-4 phényl-6 pyridazine et on agite pendant 24 heures à température ambiante.

On évapore à siccité et reprend le résidu par l'eau et alcalinise avec du carbonate de potassium. On extrait avec de l'acétate d'éthyle et sèche la solution sur sulfate de sodium. On évapore le solvant à siccité et chromatographie sur colonne de silice.

En éluant avec de l'acétate d'éthyle, on obtient le produit attendu.

F : 120°C Rendement 61 %.

B) SR 95950 A

On porte au reflux pendant 15 heures la solution dans le méthanol de 0,5 g de l'imidazo [1,2-b] pyridazine préparée ci-dessus, 0,58 ml de solution aqueuse de formaldéhyde à 37 % et 0,416 g de morpholine.

On rajoute alors 0,58 ml de solution formaldéhyde et 0,59 g de chlorhydrate de morpholine et porte à nouveau au reflux pendant 24 heures. On évapore à siccité et reprend dans l'eau.

On extrait avec de l'acétate d'éthyle, sèche la solution sur sulfate de sodium et évapore le solvant. Le résidu cristallise et on le recristallise dans l'isopropanol

F : 134°C Rendement 78 %.

Dichlorhydrate

On dissout la base dans le minimum d'isopropanol chaud et ajoute 2,2 équivalents d'acide chlorhydrique concentré.

Après refroidissement, on essore les cristaux et lave avec de l'éther isopropylique. On recristallise dans le méthanol

F : 260°C.

EXEMPLES 11 à 16

A) En opérant comme dans l'exemple 10A mais en faisant varier l'amino pyridazine de départ, on obtient les imidazo [1,2-b] pyridazines figurant dans le tableau 3.

TABLEAU 3

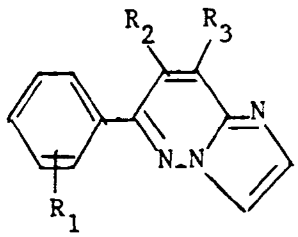

| $R_1$ | $R_2$ | $R_3$ | Point de fusion ou CCM |
|---|---|---|---|
| H | H | H | F : 112°C |
| H | H | 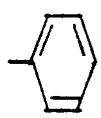 | Huile - Rf : 0,75 (acétate d'éthyle - méthanol 8/2) |
| 4-Cl | H | H | F : 160°C |
| $3\text{-}OCH_3$ | H | $CH_3$ | Huile - Rf : 0,6 (acétate d'éthyle - méthanol 95/5 à 2 % d'ammoniaque) |

A partir des imidazo [1,2-b] pyridazines décrits dans l'exemple 10A et dans le tableau 3, on obtient suivant la technique de l'exemple 10B et en faisant varier l'amine utilisée, les composés Ib réunis dans le tableau 4.

Dans le cas particulier des dérivés où $R_4 = R_5 = CH_3$, on peut effectuer la réaction par chauffage avec l'iodure de diméthyl méthylène ammonium (Sel d'Eschenmoser) au sein du chloroforme.

TABLEAU 4

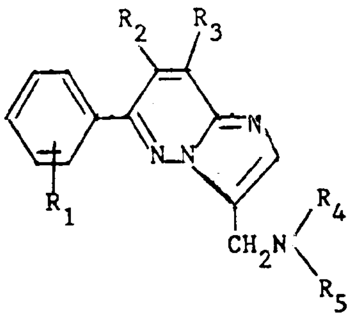

| Ex n° | N° Code SR | $R_1$ | $R_2$ | $R_3$ | $-N$ $R_4$ $R_5$ | Sel de Base Point de fusion |
|---|---|---|---|---|---|---|
| 11 | 96111 A | H | H | $-C_6H_5$ | 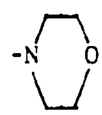 | Monochlorhydrate, 0,5$H_2O$ F : 240°C (éthanol) |
| 12 | 96109 A | 4-Cl | H | H | " " | Monochlorhydrate,1,5$H_2O$ F : 230°C (éthanol) |
| 13 | 96088 A | 3-$OCH_3$ | H | $CH_3$ | " " | Dichlorhydrate,1$H_2O$ F : 237°C (méthanol) |
| 14 | 96071 A | H | H | $CH_3$ | $-N$ $CH_3$ $CH_3$ | Dichlorhydrate, 1,5$H_2O$ F : 251°C |
| 15 | 96070 A | H | H | H | " " | Base F : 80°C Dichlorhydrate,3$H_2O$ F : 234°C |
| 16 | 96068 A | H | H | H | 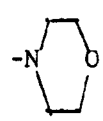 | Base F : 114°C Dichlorhydrate,2$H_2O$ F : 200°C |

Les produits selon l'invention ont été étudiés en ce qui concerne leur action thérapeutique. Notamment l'intéraction des produits selon l'invention vis-à-vis des récepteurs muscariniques cholinergiques a été déterminée.

Chez les mammifères, il existe deux sous-classes de récepteurs cholinergiques muscariniques : les récep-

teurs $M_1$ et $M_2$.

Les récepteurs de type $M_1$ sont concentrés dans certaines zones du cerveau telles que l'hippocampe, le cortex cérébral, le striatum ainsi que dans les ganglions sympathiques. Ces sites de liaison peuvent être sélectivement marqués par la [$^3$H] pirenzépine ([$^3$H] PZ). Ceux de type $M_2$ prédominent dans le coeur et dans l'iléon et peuvent être marqués par le [$^3$H] N-méthylscopolamine ([$^3$H] NMS). Afin de déterminer la sélectivité des composés de l'invention, vis-à-vis des sites $M_1$ et $M_2$, on a étudié leur intéraction in vitro avec les fixations à haute affinité de la [$^3$H] Pz et de la [$^3$H] NMS sur des membranes d'hippocampe de rat et de muscle lisse d'iléon de cobaye, respectivement.

## METHODOLOGIES

A) Recherche d'une affinité pour le récepteur cholinergique muscarinique de type $M_1$ .

L'intéraction des molécules avec les récepteurs muscariniques de type $M_1$ a été étudiée en mesurant in vitro sur un homogénat d'hippocampe de rat, le déplacement de la pirenzépine tritiée ([$^3$H] PZ) de ses sites de fixation spécifiques. Des aliquotes (10 µl) d'un homogénéisat d'hippocampe de rat à 5 % (P/v) dans un tampon $Na_2HPO_4$ (50 mM, pH 7,40), sont incubés 2 h à 4°C en présence de [$^3$H] PZ (76 Ci/nmole ; 1 nM final) et de concentrations croissantes en produit à étudier. Le volume final est de 2 ml. La réaction est arrêtée par centrifugation 10 min à 50.000 xg. Après décantation et lavage des culots, la radioactivité fixée est comptée par scintillation liquide. La fixation non spécifique est déterminée en présence de 10 µM de sulfate d'atropine. La concentration inhibitrice 50 ($CI_{50}$) est déterminée graphiquement. (Réf. : Watson J.D., Roeskoe W.R. and Yamamura H.I., Life Sci., 31, 2019-2029, 1982).

B) Recherche d'une affinité pour le récepteur cholinergique muscarinique de type $M_2$

L'intéraction avec les récepteurs muscariniques de type $M_2$ a été étudiée en mesurant in vitro sur un homogénat de muscle lisse d'iléon de cobaye, le déplacement de la N-méthyl-scopolamine tritiée ([$^3$H] NMS) de ses sites de fixation spécifiques. Des aliquots (50 µl) d'un homogénéisat de muscle lisse d'iléon de cobaye à 0,625 % (P/v) dans du tampon MEPES (20 mM) contenant NaCl (100 mM) et Mg $Cl_2$ (10 mM) (pH final : 7,5), sont incubés 20 min à 30°C en présence de [$^3$H] NMS (85 Ci/nmole ; 0,3 nM final) et de concentrations croissantes en produits à tester. Le volume final est de 1 ml. La réaction est arrêtée par centrifugation 5 min à 15.000 xg. La fixation non spécifique est déterminée en présence de 10 µM de sulfate d'atropine.

(Réf. : Hammer R., Berrie C.P., Birdsall N.I.M., Burgen A.S.V. and Hulme E.C., Nature, 283, 90-92, 1980.
Hulme E.C., Birdsall N.I.M., Burgen A.S.V. and Mettha P., Mol. Pharmacol., 14,737-750, 1978).

C) RESULTATS

Le tableau 5 indique les affinités des produits de l'invention pour les récepteurs $M_1$ et $M_2$ . Les résultats sont exprimés en concentrations inhibitrices 50 pour cent (CI 50), soit la concentration (en µM) qui induit un déplacement de 50 % du ligand tritié fixé aux récepteurs membranaires. La $CI_{50}$ de déplacement de la $^3$H-pirenzépine représente l'affinité pour le récepteur $M_1$ ; la $CI_{50}$ de déplacement de la $^3$H-NMS représente l'affinité pour le récepteur $M_2$.

De plus dans la 3e colonne on a indiqué le rapport r entre les $CI_{50}$ $M_1$ et $M_2$ qui exprime la sélectivité des produits vis-à-vis de l'un des types de récepteurs.

TABLEAU 5

| Produit n° | $^3$H-Pirenzépine ($M_1$) $CI_{50}$ µM | $^3$H-NMS ($M_2$) $CI_{50}$ µM | r= ($M_2/M_1$) |
|---|---|---|---|
| SR 95950 A | 1,4 | >100 | >71 |
| SR 96052 A | 0,5 | 65 | 130 |
| SR 96069 A | 1,5 | 100 | 66 |
| SR 96088 A | 8 | n.d. | - |
| SR 96089 A | 3 | >100 | >33 |
| SR 96095 A | 0,4 | 65 | 162 |

n.d. : non déterminé

Ces résultats montrent que les composés selon l'invention présentent une forte affinité pour les récepteurs cholinergiques muscariniques avec une spécifité marquée pour les récepteurs centraux de type $M_1$.

Par ailleurs certains des composés selon l'invention ont été soumis à une étude pharmacologique in vivo.

ETUDE PHARMACOLOGIQUE IN VIVO

La pirenzépine (PZ) est un antagoniste spécifique des récepteurs cholinergiques muscariniques centraux $M_1$. L'injection intrastriatale de PZ chez la souris induit un comportement rotatoire. On a étudié l'antagonisme de ce comportement par les produits selon l'invention.

Les produits selon l'invention sont injectés par voie intrapéritonéale (i.p.) après avoir été solubilisés dans l'eau distillée ou mis en suspension dans une solution de gomme arabique à 5 %. Les contrôles sont réalisés par injection du solvant pur dans les mêmes conditions.

Les animaux utilisés sont des souris femelles (Swiss, CD 1, Charles River, France) de poids corporel compris entre 25 et 30 grammes.

La pirenzépine est mise en solution dans un tampon phosphate, le pH de la solution est 6.

Les produits à étudier ou leurs solvants sont injectés par voie i.p. sous un volume de 0,4 ml pour 20 g de poids corporel, 15 minutes avant une injection directe de pirenzépine à la dose de 1µg dans 1 µl de solvant dans le striatum droit de la souris, selon la méthode décrite par P. WORMS et al. dans Eur. J. Pharmacol., 1986, 121, 395-401.

Le nombre de rotations contralatérales (sens opposé au côté de l'injection) a été compté pendant trois périodes de 2 minutes après injection de pirenzépine : minutes 2 à 4, 8 à 10 et 13 à 15. Chaque traitement comporte 3 à 4 doses et 10 animaux par dose. Pour chaque traitement, on calcule le nombre total de rotations et le pourcentage d'antagonisme vis-à-vis du lot contrôle.

Pour chaque produit on détermine graphiquement la dose efficace 50 ($DE_{50}$) : dose qui diminue de 50 % le nombre de rotations induites par la pirenzépine. Les résultats sont reportés dans le tableau 6.

TABLEAU 6

| Produits n° | Antagonisme Pirenzépine DE50 mg/kg i.p. |
|---|---|
| SR 95842 A | 3 |
| SR 95950 A | 5 |
| SR 96052 A | 8 |
| SR 96068 A | 10 |
| SR 96070 A | 3 |
| SR 96095 A | 5 |
| SR 96109 A | 8 |
| SR 96111 A | 7 |

Par ailleurs des essais effectués sur quelques uns des composés selon l'invention ont mis en évidence que les composés (I) franchissent les barrières hématoencéphalique et digestive.

Enfin les composés selon l'invention n'ont manifesté aucun signe de toxicité apparente aux doses où ils sont actifs.

Par suite les composés (I) peuvent être utilisés en tant que médicaments et notamment dans les cas où se manifeste un déficit cholinergique cortical et en particulier dans le cas des démences de type Alzheimer.

En conséquence selon un autre de ses aspects la présente demande concerne les compositions pharmaceutiques contenant au moins un des composés de formule (I) ou un de leurs sels en tant qu'ingrédient actif.

Dans les compositions pharmaceutiques de la présente invention pour l'administration orale, sublinguale, sous-cutanée, intramusculaire, intraveineuse, transdermique ou rectale, les ingrédients actifs de formule I ci-dessus peuvent être administrés sous formes unitaires d'administration, en mélange avec des supports pharmaceutiques classiques, aux êtres humains notamment pour le traitement des démences séniles. Les formes unitaires d'administration appropriées comprennent les formes par voie orale telles que les comprimés, les gélules, les poudres, les granulés et les solutions ou suspensions orales, les formes d'administration sublinguale et buccale, les formes d'administration sous-cutanée, intramusculaire ou intraveineuse et les formes d'administration rectale.

Afin d'obtenir l'effet désiré, la dose de principe actif peut varier entre 50 et 2000 mg par jour.

Chaque dose unitaire peut contenir de 10 à 500 mg d'ingrédient actif en combinaison avec un support pharmaceutique. Cette dose unitaire peut être administrée 1 à 4 fois par jour.

Lorsqu'on prépare une composition solide sous forme de comprimés, on mélange l'ingrédient actif principal avec un véhicule pharmaceutique tel que la gélatine, l'amidon, le lactose, le stéarate de magnésium, le talc, la gomme arabique ou analogues. On peut enrober les comprimés de saccharose ou d'autres matières appropriées ou encore on peut les traiter de telle sorte qu'ils aient une activité prolongée ou retardée et qu'ils libèrent d'une façon continue une quantité prédéterminée de principe actif.

On obtient une préparation en gélules en mélangeant l'ingrédient actif avec un diluant et en versant le mélange obtenu dans des gélules molles ou dures.

Les poudres ou les granulés dispersibles dans l'eau peuvent contenir l'ingrédient actif en mélange avec des agents de dispersion ou des agents mouillants, ou des agents de mise en suspension, comme la polyvinylpyrrolidone, de même qu'avec des édulcorants ou des correcteurs du goût.

Pour une administration rectale, on recourt à des suppositoires qui sont préparés avec des liants fondant à la température rectale, par exemple du beurre de cacao ou des polyéthylèneglycols.

Pour une administration parentérale, on utilise les suspensions aqueuses, des solutions salines isotoniques ou solutions stériles et injectables qui contiennent des agents de dispersion et/ou des mouillants pharmacologiquement compatibles, par exemple le propylèneglycol ou le butylèneglycol.

Le principe actif peut être formulé également sous forme de microcapsules, éventuellement avec un ou plusieurs supports ou additifs.

Ainsi à titre d'exemple, on peut préparer des gélules à base d'un des composés des exemples 1 à 16 ayant la composition suivante :

| | |
|---|---|
| Principe actif | 25 mg |
| Lactose | 110 mg |
| Stéarate de magnésium | 5mg |

en mélangeant intimement les ingrédients ci-dessus et en versant le mélange dans des gélules de gélatine dure.

## Revendications

1. Imidazo[1,2-b]pyridazines de formule générale :

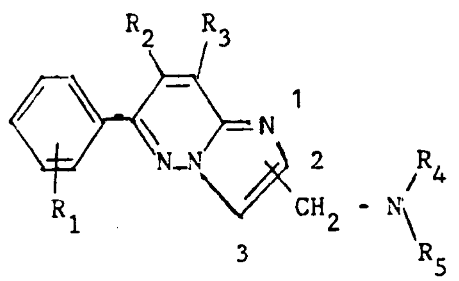

(I)

dans laquelle

$R_1$ représente l'hydrogène, un atome d'halogène, un groupe alcoxy en $C_1$ - $C_4$, un groupe alkyle en $C_1$ - $C_4$ occupant l'une des positions libres du noyau benzénique ;

$R_2$ représente l'hydrogène, un groupe alkyle en $C_1$ - $C_4$ ou un groupe phényle ;

$R_3$ représente l'hydrogène, un groupe alkyle en $C_1$ - $C_4$ ou un groupe phényle ;

le groupe

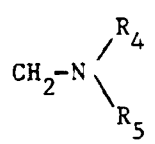

occupe la position 2 ou 3 du cycle et dans ce groupe $R_4$ et $R_5$ considérés indépendemment représentent l'hydrogène ou un groupe alkyle en $C_1$ - $C_4$ ou encore $R_4$ et $R_5$ considérés avec l'atome d'azote auquel ils sont liés constituent un hétérocycle à 5 ou 6 chaînons contenant éventuellement un second hétéroatome,
ainsi que leurs sels avec les acides minéraux ou organiques pharmaceutiquement acceptables.

2. Composés selon la revendication 1 dans lesquels le groupe

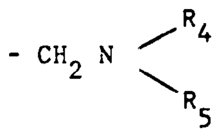

occupe la position 2 du cycle imidazolique et où $R_1$, $R_2$, $R_3$, $R_4$ et $R_5$ sont tels que définis dans la revendication 1.

3. Composés selon la revendication 1 dans lesquels le groupe

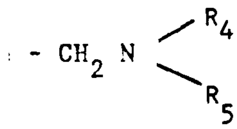

occupe la position 3 du cycle imidazolique et où $R_1$, $R_2$, $R_3$, $R_4$ et $R_5$ sont tels que définis dans la revendication 1.

4. Procédé de préparation des composés de formule (I) selon la revendication 2, caractérisé en ce que :

– on chauffe une amino-3 phényl-6 pyridazine convenablement substituée, avec de la 1,3-di-chloracétone au sein d'un solvant comme le dioxanne ou l'acétonitrile pendant 12 à 15 heures, puis on ajoute de l'acétate de sodium et poursuit le chauffage pendant 2 à 5 heures pour former une chlorométhyl-2 phényl-6 imidazo pyridazine de formule :

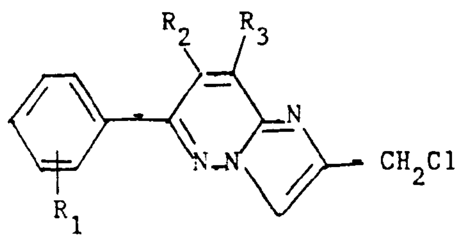

– on chauffe le dérivé ainsi obtenu avec une amine

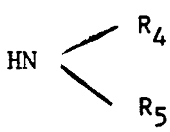

au sein d'un solvant inerte pour former le composé (I) correspondant

– éventuellement on salifie le composé (I) ainsi obtenu.

5. Procédé de préparation des composés de formule (I) selon la revendication 3, caractérisé en ce que :

– on traite entre 20 et 50°C une amino-3 phényl-6 pyridazine convenablement substituée par un bromacétaldéhyde en solution dans un solvant protique pour former une phényl-6 imidazo pyridazine de formule :

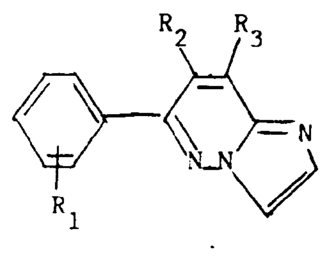

– on substitue celle-ci par action du formol et d'une amine

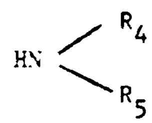

pour obtenir les composés de formule (I) correspondants.

– éventuellement, on salifie les composés (I) ainsi obtenus.

6. Compositions pharmaceutiques contenant à titre de principe actif un composé de formule (I) selon revendication (I) en combinaison avec un véhicule pharmaceutiquement acceptable.

## Patentansprüche

1. Imidazo-[1,2-b]-pyridazine der allgemeinen Formel

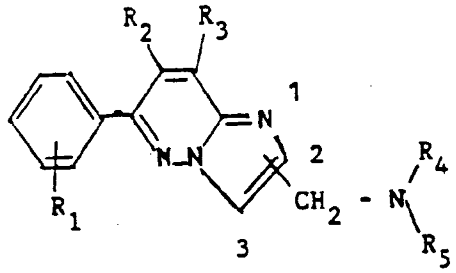

(I)

worin

$R_1$ Wasserstoff, ein Halogenatom eine $C_1$-$C_4$-Alcoxygruppe, eine $C_1$-$C_4$-Alkylgruppe, die eine der freien Positionen des Benzolkerns einnimmt, darstellt;

$R_2$ Wasserstoff, eine $C_1$-$C_4$-Alkylgruppe oder eine Phenylgruppe darstellt;

$R_3$ Wasserstoff, eine $C_1$-$C_4$-Alkylgruppe oder eine Phenylgruppe darstellt;

die Gruppe

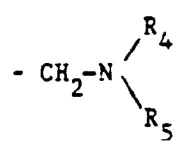

die Position 2 oder 3 des Ringes einnimmt und in dieser Gruppe $R_4$ und $R_5$, einzeln betrachtet, Wasserstoff oder eine $C_1$-$C_4$-Alkylgruppe darstellen, oder aber $R_4$ und $R_5$, mit dem Stickstoffatom an das sie gebunden sind, betrachtet, einen Heterocyclus mit 5 oder 6 Ketten, der gegebenenfalls ein zweites Heteroatom enthält, bilden, sowie die pharmazeutisch akzeptablen Salze derselben mit Mineral- oder organischen Säuren.

2. Verbindungen nach Anspruch 1, in denen die Gruppe

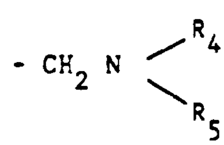

die Position 2 des Imidazolringes einnimmt, und worin $R_1$, $R_2$, $R_3$, $R_4$ und $R_5$ wie in Anspruch 1 definiert sind.

3. Verbindungen nach Anspruch 1, in denen die Gruppe

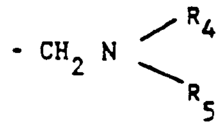

die Position 3 des Imidazolringes einnimmt, und worin $R_1$, $R_2$, $R_3$, $R_4$, und $R_5$ wie in Anspruch 1 definiert sind.

4. Verfahren zur Herstellung der Verbindungen der Formel (I) nach Anspruch 2, dadurch gekennzeichnet, daß

– ein zweckmäßig substituiertes 3-Amino-6-phenylpyridazin mit 1,3-dichloraceton in einem Lösungsmittel, wie Dioxan oder Acetonitril, während 12 bis 15 Stunden erhitzt wird, danach Natriumacetat zugegeben und das Erhitzen während 2 bis 5 Stunden fortgesetzt wird, um ein 2-Chlormethyl-6-phenyl-imidazopyridazin der Formel

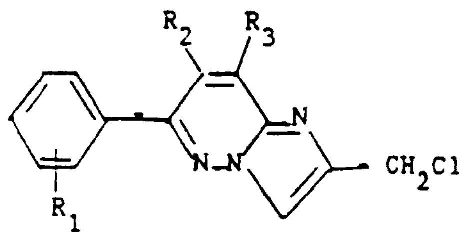

zu bilden,

– das so erhaltene Derivat mit einem Amin

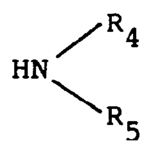

in einem inerten Lösungsmittel zur Bildung der entsprechenden Verbindung (I) erhitzt wird,

– gegebenenfalls die so erhaltene Verbindung (I) in das Salz übergeführt wird.

5. Verfahren zur Herstellung der Verbindungen der Formel (I) nach Anspruch 3, dadurch gekennzeichnet, daß

– ein zweckmäßig substituiertes 3-Amino-6-phenylpyridazin zwischen 20 und 50°C mit einem Bromacetaldehyd in Lösung in einem protischen Lösungsmittel behandelt wird, um ein 6-phenyl-imidazopyridazin der Formel

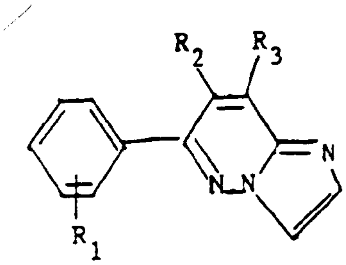

zu bilden;

– dieses durch Einwirken von Formol und eines Amins

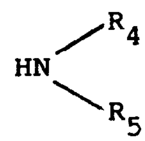

substituiert wird, um die entsprechenden Verbindungen der Formel (I) zu erhalten,

– gegebenenfalls die so erhaltenen Verbindungen (I) in Salze übergeführt werden.

6. Pharmazeutische Zusammensetzungen, die als Wirkstoff eine Verbindung der Formel (I) nach Anspruch 1 in Kombination mit einem pharmazeutisch akzeptablen Vehikel enthalten.

## Claims

1. Imidazo[1,2-b]pyridazines of the general formula:

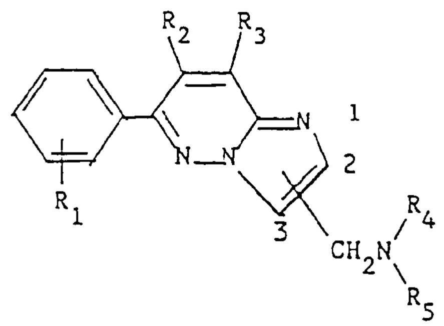

(I)

in which:

$R_1$ represents hydrogen, a halogen atom, a $C_1$-$C_4$ alkoxy group or a $C_1$-$C_4$ alkyl group occupying one of the free positions of the benzene ring;

$R_2$ represents hydrogen, a $C_1$-$C_4$ alkyl group or a phenyl group;

$R_3$ represents hydrogen, a $C_1$-$C_4$ alkyl group or a phenyl group; and

the group

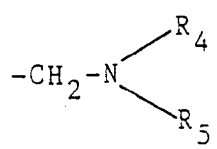

occupies the 2-position or 3-position of the ring and, in this group, $R_4$ and $R_5$, taken independently, represent hydrogen or a $C_1$-$C_4$ alkyl group, or $R_4$ and $R_5$, taken with the nitrogen atom to which they are bonded, form a 5-membered or 6-membered heterocycle optionally containing a second heteroatom,

and their salts with pharmaceutically acceptable mineral or organic acids.

2. Compounds according to claim 1 in which the group

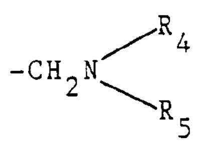

occupies the 2-position of the imidazole ring and in which $R_1$, $R_2$, $R_3$, $R_4$ and $R_5$ are as defined in claim 1.

3. Compounds according to claim 1 in which the group

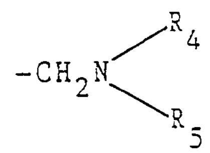

occupies the 3-position of the imidazole ring and in which $R_1$, $R_2$, $R_3$, $R_4$ and $R_5$ are as defined in claim 1.

4. Process for the preparation of the compounds of formula (I) according to claim 2, characterized in that

– an appropriately substituted 3-amino-6-phenylpyridazine is heated with 1,3-dichloroacetone in a solvent such as dioxane or acetonitrile, for 12 to 15 hours, sodium acetate is then added and heating is continued for 2 to 5 hours to form a 2-chloromethyl-6-phenylimidazopyridazine of the formula:

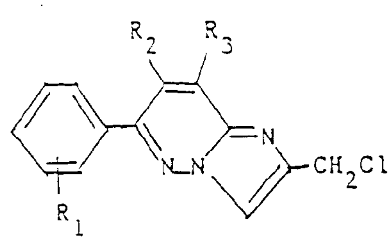

;

– the resulting derivative is heated with an amine

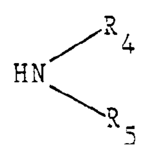

in an inert solvent to form the corresponding compound (I); and

– if desired, the resulting compound (I) is salified.

5. Process for the preparation of the compounds of formula (I) according to claim 3, wherein:

– an appropriately substituted 3-amino-6-phenylpyridazine is treated, at between 20 and 50°C, with bromoacetaldehyde in solution in a protic solvent to form a 6-phenylimidazopyridazine of the formula:

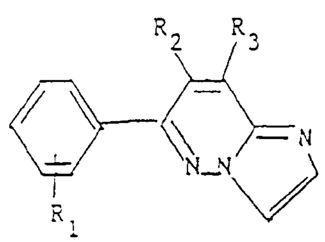

;

– this is substituted by reaction with formaldehyde solution and an amine

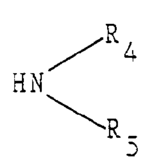

to give the corresponding compounds of formula (I); and

– if desired, the resulting compounds (I) are salified.

6. Pharmaceutical compositions in which a compound of formula (I) according to claim 1 is present as the active principle, in combination with a pharmaceutically acceptable vehicle.